# EUROPEAN PATENT SPECIFICATION

(11) **EP 4 030 788 B1**
(45) Date of publication and mention of the grant of the patent: **03.09.2025**
(21) Application number: 21152083.8
(22) Date of filing: 18.01.2021
(51) Int. Cl.: H04W 4/02, H04W 4/029, G06F 21/62, G16H 50/80, G08B 21/22

(54) **METHOD FOR SECURELY REVEALING A PERSONAL IDENTIFIER OF OR RELATED TO TWO PERSONS, RESPECTIVELY, TO A CONTACT TRACING ENTITY OF A CONTACT TRACING SYSTEM OR METHOD, SYSTEM, COMPUTING DEVICE OR FUNCTIONALITIES AND WEARABLE OR PORTABLE DEVICES, PROGRAM AND COMPUTER-READABLE MEDIUM**
VERFAHREN ZUR SICHEREN PREISGABE EINES PERSÖNLICHEN IDENTIFIKATORS VON ODER IN BEZUG AUF ZWEI PERSONEN AN EINE KONTAKTVERFOLGUNGSEINHEIT EINES KONTAKTVERFOLGUNGSSYSTEMS ODER -VERFAHRENS, SYSTEM, RECHNERVORRICHTUNG ODER FUNKTIONALITÄTEN UND WEARABLE- ODER TRAGBARE VORRICHTUNGEN, PROGRAMM UND COMPUTERLESBARES MEDIUM
PROCÉDÉ PERMETTANT DE RÉVÉLER DE MANIÈRE SÛRE UN IDENTIFIANT PERSONNEL DE OU LIÉ À DEUX PERSONNES, RESPECTIVEMENT, À UNE ENTITÉ DE RECHERCHE DE CONTACTS D'UN SYSTÈME OU D'UN PROCÉDÉ DE RECHERCHE DE CONTACTS, SYSTÈME, DISPOSITIF OU FONCTIONNALITÉS INFORMATIQUES ET DISPOSITIFS PORTABLES OU PORTATIFS, PROGRAMME ET SUPPORT LISIBLE PAR ORDINATEUR

(43) Date of publication of application: 20.07.2022
(73) Proprietor: Deutsche Telekom AG, 53113 Bonn (DE)
(72) Inventor: ROLLIN, Hannes, 19055 Schwerin (DE); QUECK, Oliver, 72813 Sankt Johann - Würtingen (DE)
(74) Representative: Schwöbel, Thilo K.

(56) References cited:
- DE-U1- 202020 103 175
- WILLIAM J BUCHANAN ET AL: "Review and Critical Analysis of Privacy-preserving Infection Tracking and Contact Tracing", ARXIV.ORG, CORNELL UNIVERSITY LIBRARY, 201 OLIN LIBRARY CORNELL UNIVERSITY ITHACA, NY 14853, 10 September 2020 (2020-09-10), XP081760207
- "Comparison of existing pandemic contact tracing systems", vol. ISG - E4P, no. V1.1.1, 17 December 2020 (2020-12-17), pages 1 - 113, XP014396414, Retrieved from the Internet <URL:ftp://docbox.etsi.org/ISG/E4P/Open/e4p-002v111publishedversion_v120internalversion.pdf> [retrieved on 20201217]

## Description

### BACKGROUND

The present invention relates to a method for securely revealing a personal identifier of or related to two persons, respectively, to a contact tracing entity of a contact tracing system or method based on detecting relevant encounters between two persons, wherein each of the plurality of persons are wearing or carrying a wearable or portable device, the wearable or portable devices having or being associated or assigned to, respectively, a tag identifier.

Furthermore, the present invention relates to a system for securely revealing a personal identifier of or related to two persons, respectively, to a contact tracing entity of a contact tracing system or method based on detecting relevant encounters between two persons, wherein each of the plurality of persons are wearing or carrying a wearable or portable device, the wearable or portable devices having or being associated or assigned to, respectively, a tag identifier.

Additionally, the present invention relates to computing device or functionalities and wearable or portable devices for securely revealing a personal identifier of or related to two persons, respectively, to a contact tracing entity of a contact tracing system or method based on detecting relevant encounters between two persons, wherein each of the plurality of persons are wearing or carrying a wearable or portable device, the wearable or portable devices having or being associated or assigned to, respectively, a tag identifier

Additionally, the present invention relates to a program and to a computer-readable medium for securely revealing a personal identifier of or related to two persons, respectively, to a contact tracing entity of a contact tracing system or method according to the inventive method.

It is conventionally known to realize contact detection and/or contact tracing for a plurality of persons by means of using wearable or portable devices, especially so-called ultra-wideband wearable or portable devices, or ultra-wideband tags. Such wearable or portable devices typically store log data related to the contact situation of the respective or considered wearable or portable device with other wearable or portable devices being or having been in proximity. Such log data typically comprise information about possibly risky encounters between at least two persons. In case such two persons are prepared to reveal at least parts of their log data, such log data might nevertheless comprise relevant information regarding other persons, not having agreed to reveal their corresponding data. Non-patent literature "Review and Critical Analysis of Privacy-preserving Infection Tracking and Contact Tracing", DE202020103175U U1 20200618 and non-patent literature "ETSI GR E4PO-002 V1.1. - published version-V.1.2.0 internal version (2020-11) Comparison of existing pandemic contact tracing systems" represent prior art in the field of pandemic contact tracing.

### SUMMARY

An object of the present invention is to provide a technically simple, effective and cost effective solution for securely revealing a personal identifier of or related to two persons, respectively, to a contact tracing entity of a contact tracing system or method based on detecting relevant encounters between two persons, wherein each of the plurality of persons are wearing or carrying a wearable or portable device, the wearable or portable devices having or being associated or assigned to, respectively, a tag identifier, the tag identifiers being repeatedly broadcast by the wearable or portable devices, respectively, using a wireless communication interface of the wearable or portable devices. A further object of the present invention is to provide a corresponding system, computing devices or functionalities and wearable or portable devices, and a corresponding program and computer-readable medium.

The object of the present invention is achieved by a method for securely revealing a personal identifier of or related to two persons, respectively, to a contact tracing entity of a contact tracing system or method based on detecting relevant encounters between two persons, wherein each of the plurality of persons are wearing or carrying a wearable or portable device, the wearable or portable devices having or being associated or assigned to, respectively, a tag identifier, the tag identifiers being repeatedly broadcast by the wearable or portable devices, respectively, using a wireless communication interface of the wearable or portable devices, thereby generating and/or storing log data from which, respectively, encounter event related data and further encounter event related data are able to be derived, comprising, respectively, both tag identifiers, a computing device or functionality being associated or related to the wearable or portable device, as well as a further computing device or functionality being associated or related to the further wearable or portable device,
wherein a hash function is used for securely revealing the personal identifiers in case that the concerned persons are both opting-in for such revealing, the method comprises the following steps:
-- in a first step, a relevant encounter of the wearable or portable device and the further wearable or portable device is detected, independently from each other, by the wearable or portable device and the further wearable or portable device, thereby generating and transmitting
   -- the corresponding encounter event related data, comprising both tag identifiers as well as at least one encounter time stamp information, to the computing device or functionality, and
   -- the corresponding further encounter event related data, comprising both tag identifiers as well as at least one further encounter time stamp information, to the further computing device or functionality,
-- in a second step, the computing device or functionality generates upload data and the further computing device or functionality generates further upload data, both the computing device or functionality and the further computing device or functionality transmitting the respective upload data to the contact tracing entity, wherein
   -- the computing device or functionality, upon being informed about a diagnosis, especially a positive test result, generates a diagnosis hash value by computing the hash function from a diagnosis hash input information, comprising both tag identifiers in a diagnosis order, especially the wearable or portable device's tag identifier first, the diagnosis hash value as well as the personal identifier being part of the upload data, and
   -- the further computing device or functionality generates a further hash value by computing the hash function from a hash input information comprising both tag identifiers in a standard order, especially the wearable or portable device's tag identifier first, the further hash value as well as the further personal identifier being part of the further upload data,
resulting in the contact tracing entity being able to know, by means of both the diagnosis hash value and the further hash value being identical, about the relevant encounter of the wearable or portable device and the further wearable or portable device as well as of the personal identifiers.

It is thereby advantageously possible according to the present invention to effectively and securely reveal the personal identifiers, to a contact tracing entity (such as, e.g., a or connected to a human resources department of an enterprise), of the persons (or users) having been in comparatively close contact, provided these persons and users have opted in for such revealing.

When using contact detecting and/or tracing tags or wearable or portable devices, these tags or wearable or portable devices allow to combine high-precision contact tracing - especially if ultra-wideband, UWB, wearables or wearable devices or tags are used - especially with a decentralized risk calculation on a computing device, often or typically the users' smartphones. Typically, each wearable or portable device or tag has a unique ID, or tag identifier which is broadcast and saved by other wearable or portable devices or tags in the vicinity. The sum or combination of these measurements gives rise to logs (or log data), which are transported to a computing device or functionality, often a user's smart phone or other personal digital assistant, or part of the wearable or portable device, where the log data must be processed to detect risk encounters.

The wearable or portable devices might either not be personalized per se (and in this case they can be used and reused by anyone (typically among the group of persons concerned, e.g. the staff of an organization or a differently defined group of persons)), but during one session, only a single user is typically using one wearable device or tag. According to another embodiment of the present invention, the wearable or portable device is able to provide the functionality of both a tag (or wearable device, i.e. primarily directed to contact detection) and of a computing device or functionality, i.e. for analyzing the log data.

According to the present invention, normally a certain number of persons is considered such as, especially, the staff of an enterprise or of an organization. It is aimed at enabling contact detection and contact tracing for these persons such that contact tracing is able to be performed individually for each person, and typically performed by each person individually using their personal computing device, especially a smart phone or other personal portable device. However, as the wearable or portable device does not necessarily be a dedicated contact tracking or detecting device but might also be embodied as, e.g., a mobile phone of the person wearing or carrying it, the number of persons is not necessarily strictly limited a priori to, e.g., the staff of an enterprise or of an organization.

To each wearable or portable device is assigned or each wearable or portable device is associated to a tag identifier, i.e. an identifying information such as a serial number or the like. At a specific point in time, each wearable or portable device or each tag is associated to exactly one tag identifier; however, in principle according to the present invention, it is not excluded that one and the same wearable or portable device or tag is able to be associated or assigned (successively) to more than one wearable or portable device, especially in order to still enhance the level of data protection or data privacy, especially by avoiding to create specific pattern, e.g. regarding the usage of certain wearable or portable devices or tags by certain persons or the like. However, in case that more than one tag identifiers are able to be associated or assigned to one and the same wearable or portable device, a mechanism is needed to securely identify or determine such tag identifiers without doubt each time the wearable or portable device is used. In the following, the invention is mainly described under the assumption that a tag identifier is fixedly, i.e. unchangeably, associated or assigned to a wearable or portable device.

According to the present invention, the wearable or portable devices, while in operation, more or less continuously, i.e. repeatedly, broadcast the assigned or associated (at this point in time unique) tag identifier (using the wireless communication interface of the wearable or portable devices), i.e. these broadcasts are typically performed, by the wearable or portable devices and under normal conditions, by applying a certain rate of broadcasts (i.e. a number of broadcasts per time unit, such as, typically once per second (i.e. 1 Hz) or the like; usual such broadcast rates might range from about 0,1 Hz (i.e. once every 10 seconds) to about 10 Hz (ten broadcasts per second)); a broadcast typically corresponding to a radio frequency signal being emitted by the respective wearable or portable device applying a certain standardized or usual emission power such that another wearable or portable device, located in proximity, is able to receive such radio frequency signal and derive or determine, from some signal parameter (such as, typically, some signal strength parameter) and/or from some signal content (such as, e.g., time stamp information and/or emission power information) an indication regarding the distance between both wearable or portable devices. Of course, a corresponding broadcast and radio frequency signal reception procedure is symmetric and both are occurring simultaneously. This also means that, in case that more than two wearable or portable devices are present or located at a specific location or within a considered area (of a size or extension smaller than the radio frequency coverage area of each of the wearable or portable devices), there might be many broadcasts and many radio frequency signals to be received and processed, often leading to - in case of a density of wearable or portable devices (and/or in case of a rate of radio frequency signals to be received and/or processed by each one of the wearable or portable devices considered) exceeding a certain density threshold - the wearable or portable devices broadcasting the radio frequency signals less often in such high density conditions, i.e. the above mentioned rate of broadcasts (under normal conditions) might be adaptively reduced in such high density conditions. However, each such broadcast event of a wearable or portable device involves the wearable or portable device transmitting or broadcasting its (at least temporarily) associated or assigned tag identifier (hereinafter also called tag identifier information).

Hence according to the present invention, in a contact situation of a wearable or portable device with a further wearable or portable device (of the plurality of wearable or portable devices), the wearable or portable device is able, by means of the wearable or portable device's wireless communication interface and with respect to the further wearable or portable device, to determine repeatedly the distance, at different points in time, towards the further wearable or portable device and to receive (typically at a plurality of points in time) the wearable or portable device's tag identifier. From these received broadcast pieces of information (that each wearable or portable device is able to receive from the other wearable or portable devices nearby, i.e. within the coverage area of the respective other wearable or portable device), each wearable or portable device generates (and typically stores) log data, this log data corresponding to or representing the contact situation of the wearable or portable device with the further wearable or portable device (in case of only one further wearable or portable device) or with the plurality of further wearable or portable devices (in case of more than one further wearable or portable device nearby). The log data typically comprise all distance detection events (of or measured or detected by the wearable or portable device with respect to any nearby wearable or portable devices whose radio frequency signals are detectable or receivable by the wearable or portable device), i.e. all detections of the wearable or portable device with other wearable or portable devices. For example, each contact or each distance detection event could be represented, as part of the log data by the following quadruple:
<My ID (tag identifier of the wearable or portable device), Other ID (tag identifier of the further wearable or portable device), Timestamp (the point in time of the contact or distance detection event), Distance (the distance measured or determined by the wearable or portable device)>
According to the present invention, the log data (of the wearable or portable device, i.e. the encounter event related data) are generated by the wearable or portable device (and further encounter event related data are generated by the further wearable or portable device), and transmitted to a corresponding computing device or functionality (or further computing device or functionality corresponding to the further wearable or portable device). The encounter event related data (and further encounter event related data) are eventually present at the respective computing device or functionality (typically generated by the respective computing device or functionality based on log data received from the wearable or portable devices). The encounter event related data, comprise, respectively, both tag identifiers (of the wearable or portable device and the further wearable or portable device) involved in the respective contacts or encounters.

According to the present invention, by means of using a hash function, it is advantageously possible to securely reveal (especially to a contact tracing system) the personal identifiers in case that the concerned persons are both opting-in for such revealing, the method comprises the following steps:
-- in a first step, a relevant encounter of the wearable or portable device and the further wearable or portable device is detected, independently from each other, by the wearable or portable device and the further wearable or portable device, thereby generating and transmitting
   -- the corresponding encounter event related data, comprising both tag identifiers as well as at least one encounter time stamp information, to the computing device or functionality, and
   -- the corresponding further encounter event related data, comprising both tag identifiers as well as at least one further encounter time stamp information, to the further computing device or functionality,
-- in a second step, the computing device or functionality generates upload data and the further computing device or functionality generates further upload data, both the computing device or functionality and the further computing device or functionality transmitting the respective upload data to the contact tracing entity, wherein
   -- the computing device or functionality, upon being informed about a diagnosis, especially a positive test result, generates a diagnosis hash value by computing the hash function from a diagnosis hash input information, comprising both tag identifiers in a diagnosis order, especially the wearable or portable device's tag identifier first, the diagnosis hash value as well as the personal identifier being part of the upload data, and
   -- the further computing device or functionality generates a further hash value by computing the hash function from a hash input information comprising both tag identifiers in a standard order, especially the wearable or portable device's tag identifier first, the further hash value as well as the further personal identifier being part of the further upload data,
resulting in the contact tracing entity being able to know, by means of both the diagnosis hash value and the further hash value being identical, about the relevant encounter of the wearable or portable device and the further wearable or portable device as well as of the personal identifiers.

Hence, according to the present invention, it is advantageously possible to reveal information (especially to the contact tracing system) regarding opt-in users, but to avoid that regarding non-opt-in user (or opt-out users) such personal identifier information is revealed.

According to the present invention, it is advantageously possible and preferred that, during, prior to or after the second step, the computing device or functionality generates, in the absence of an information about a diagnosis, especially a positive test result, a hash value, as part of the upload data, by computing the hash function from a hash input information, comprising both tag identifiers in the standard order, especially the further wearable or portable device's tag identifier first, resulting in the contact tracing entity being unable to know about the encounter of the wearable or portable device and the further wearable or portable device, by means of the hash value and the further hash value being different from each other.

Thereby, it is advantageously possible to provide for an efficient solution for not revealing any information in case no risk encounter has occurred.

According to the present invention, it is furthermore advantageously possible and preferred that both the upload data and the further upload data furthermore also comprises at least one piece of time stamp information, respectively, related to the relevant encounter of the wearable or portable device and the further wearable or portable device,
wherein especially the piece of time stamp information of the upload data
   -- in case of the computing device or functionality being informed about a diagnosis, corresponds to both the beginning and the end of the relevant encounter,
   -- in case of the absence of an information about a diagnosis received by the computing device or functionality, corresponds to a point in time in between the beginning and the end of the relevant encounter, as detected by the wearable or portable device, especially corresponding to the arithmetic mean of the beginning and the end of the relevant encounter, and
wherein especially the piece of time stamp information of the further upload data in case of the absence of an information about a diagnosis received by the further computing device or functionality, corresponds to a point in time in between the beginning and the end of the relevant encounter, as detected by the further wearable or portable device, especially corresponding to the arithmetic mean of the beginning and the end of the relevant encounter.

According to the present invention, it is furthermore advantageously possible and preferred that - in case that the contact tracing entity is able to know, by means of both the diagnosis hash value and the further hash value being identical, about the relevant encounter of the wearable or portable device and the further wearable or portable device - the contact tracing entity is also able to know that the at least one piece of time stamp information of both the upload data and the further upload data are corresponding to each other, especially by means of the piece of time stamp information of the further upload data indicating a point in time in between the points in time indicated by the piece of time stamp information of the upload data.

Thereby, it is advantageously possible to easily identify risk encounters, both on the basis of using the hash function on both tag identifiers and based on using the pieces of time stamp information.

According to the present invention, it is advantageously furthermore possible and preferred that both the diagnosis hash input information, and the standard hash input information comprise, besides both tag identifiers, a further information, the further information being common to both the wearable or portable device and the further wearable or portable device but continuously changing over time, the further information especially corresponding to the current date, or the current week of the relevant encounter.

Thereby, it is advantageously possible to further enhance the level of privacy or data protection regarding users of the system.

According to the present invention, it is furthermore advantageously possible and preferred that the personal identifier is or corresponds to an information relating to the identity of the respective person, especially the name or a further identity information, or to a pseudonym and/or wherein at least a part of the plurality of persons is opting-in for revealing the respective personal identifier.

According to a further preferred embodiment of the present invention, in a contact situation of the wearable or portable device with the further wearable or portable device, the wearable or portable device is able to repeatedly determine the distance, at different points in time, towards the further wearable or portable device, and vice versa, and to receive the further wearable or portable device's tag identifier by the wearable or portable device and the wearable or portable device's tag identifier by the further wearable or portable device, thereby especially generating and storing log data.

Furthermore, the present invention relates to a system for securely revealing a personal identifier of or related to two persons, respectively, to a contact tracing entity of a contact tracing system or method based on detecting relevant encounters between two persons,
the system especially comprising, besides a plurality of wearable or portable devices, the contact tracing entity, and computing device or functionalities,
wherein each of the plurality of persons are wearing or carrying a wearable or portable device, the wearable or portable devices having or being associated or assigned to,
respectively, a tag identifier, the tag identifiers being repeatedly broadcast by the wearable or portable devices, respectively, using a wireless communication interface of the wearable or portable devices, thereby generating and/or storing log data from which, respectively, encounter event related data and further encounter event related data are able to be derived, comprising, respectively, both tag identifiers, a computing device or functionality being associated or related to the wearable or portable device, as well as a further computing device or functionality being associated or related to the further wearable or portable device,
wherein a hash function is used for securely revealing the personal identifiers in case that the concerned persons are both opting-in for such revealing, the system being configured such that:
   -- a relevant encounter of the wearable or portable device and the further wearable or portable device is detected, independently from each other, by the wearable or portable device and the further wearable or portable device, thereby generating and transmitting
      -- the corresponding encounter event related data, comprising both tag identifiers as well as at least one encounter time stamp information, to the computing device or functionality, and
      -- the corresponding further encounter event related data, comprising both tag identifiers as well as at least one further encounter time stamp information, to the further computing device or functionality,
   -- the computing device or functionality generates upload data and the further computing device or functionality generates further upload data, both the computing device or functionality and the further computing device or functionality transmitting the respective upload data to the contact tracing entity, wherein
      -- the computing device or functionality, upon being informed about a diagnosis, especially a positive test result, generates a diagnosis hash value by computing the hash function from a diagnosis hash input information, comprising both tag identifiers in a diagnosis order, especially the wearable or portable device's tag identifier first, the diagnosis hash value as well as the personal identifier being part of the upload data, and
      -- the further computing device or functionality generates a further hash value by computing the hash function from a hash input information comprising both tag identifiers in a standard order, especially the wearable or portable device's tag identifier first, the further hash value as well as the further personal identifier being part of the further upload data,
resulting in the contact tracing entity being able to know, by means of both the diagnosis hash value and the further hash value being identical, about the relevant encounter of the wearable or portable device and the further wearable or portable device as well as of the personal identifiers.

Furthermore, the present invention relates to computing devices or functionalities and wearable or portable devices for securely revealing a personal identifier of or related to two persons, respectively, to a contact tracing entity of a contact tracing system or method based on detecting relevant encounters between two persons, wherein each of the plurality of persons are wearing or carrying a wearable or portable device, the wearable or portable devices having or being associated or assigned to, respectively, a tag identifier, the tag identifiers being repeatedly broadcast by the wearable or portable devices, respectively, using a wireless communication interface of the wearable or portable devices, thereby generating and/or storing, respectively, encounter event related data and further encounter event related data, comprising, respectively, both tag identifiers, a computing device or functionality being associated or related to the wearable or portable device, as well as a further computing device or functionality being associated or related to the further wearable or portable device, wherein a hash function is used for securely revealing the personal identifiers in case that the concerned persons are both opting-in for such revealing, the computing device or functionality and the wearable or portable devices being configured such that:
-- a relevant encounter of the wearable or portable device and the further wearable or portable device is detected, independently from each other, by the wearable or portable device and the further wearable or portable device, thereby generating and transmitting
   -- the corresponding encounter event related data, comprising both tag identifiers as well as at least one encounter time stamp information, to the computing device or functionality, and
   -- the corresponding further encounter event related data, comprising both tag identifiers as well as at least one further encounter time stamp information, to the further computing device or functionality,
-- the computing device or functionality generates upload data and the further computing device or functionality generates further upload data, both the computing device or functionality and the further computing device or functionality transmitting the respective upload data to the contact tracing entity, wherein
   -- the computing device or functionality, upon being informed about a diagnosis, especially a positive test result, generates a diagnosis hash value by computing the hash function from a diagnosis hash input information, comprising both tag identifiers in a diagnosis order, especially the wearable or portable device's tag identifier first, the diagnosis hash value as well as the personal identifier being part of the upload data, and
   -- the further computing device or functionality generates a further hash value by computing the hash function from a hash input information comprising both tag identifiers in a standard order, especially the wearable or portable device's tag identifier first, the further hash value as well as the further personal identifier being part of the further upload data,
resulting in the contact tracing entity being able to know, by means of both the diagnosis hash value and the further hash value being identical, about the relevant encounter of the wearable or portable device and the further wearable or portable device as well as of the personal identifiers.

Additionally, the present invention relates to a program comprising a computer readable program code which, when executed on a computer and/or on a computing device or functionality and/or on a contact tracing entity and/or on a wearable or portable device, or in part on a computing device or functionality and/or in part on a contact tracing entity and/or on a wearable or portable device, causes the computer and/or the computing device or functionality and/or the contact tracing entity and/or the wearable or portable device to perform the inventive method.

Still additionally, the present invention relates to a computer-readable medium comprising instructions which when executed on a computer and/or on a computing device or functionality and/or on a contact tracing entity and/or on a wearable or portable device, or in part on a computing device or functionality and/or in part on a contact tracing entity and/or on a wearable or portable device, causes the computer and/or the computing device or functionality and/or the contact tracing entity and/or the wearable or portable device to perform the inventive method.

These and other characteristics, features and advantages of the present invention will become apparent from the following detailed description, taken in conjunction with the accompanying drawings, which illustrate, by way of example, the principles of the invention. The description is given for the sake of example only, without limiting the scope of the invention. The reference figures quoted below refer to the attached drawings. The scope of protection sought for is defined by the appended claims.

### BRIEF DESCRIPTION OF THE DRAWINGS

Figure 1 schematically illustrates a plurality of persons in a contact detection and/or contact tracing setting, wherein each of the plurality of persons is wearing or carrying a wearable or portable device (or tag) of a plurality of wearable or portable devices (or tags).

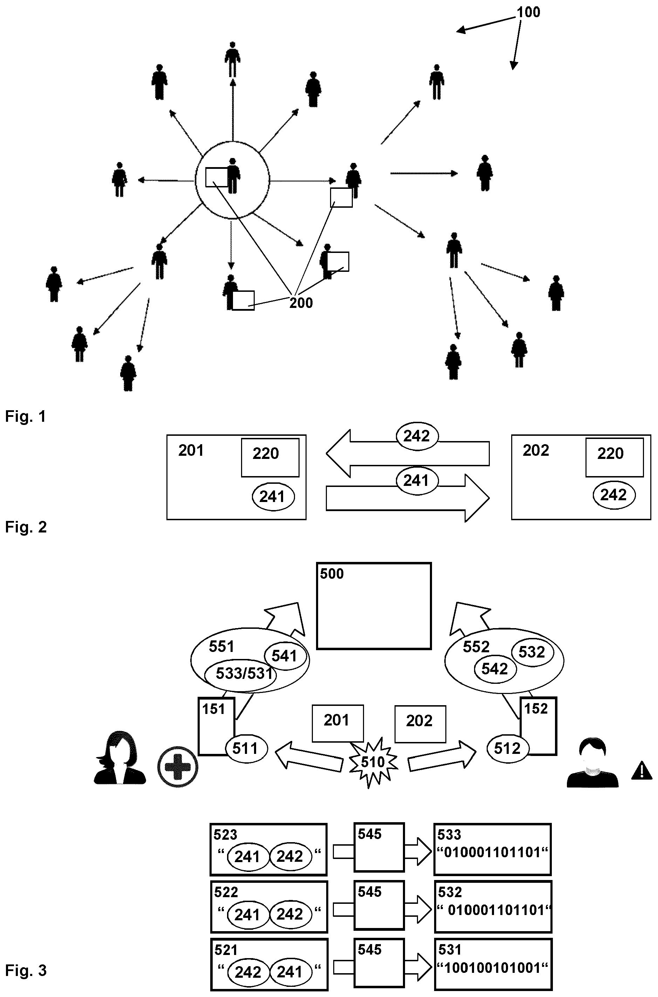

Figure 2 schematically illustrates a contact situation of a wearable or portable device with a further wearable or portable device, the wearable or portable device being able, by means of the wearable or portable device's wireless communication interface and with respect to the further wearable or portable device, to determine repeatedly the distance towards the further wearable or portable device and to receive the further wearable or portable device's tag identifier, thereby generating and storing log data of the contact situation of the wearable or portable device with the further wearable or portable device.
Figure 3 schematically illustrates an exemplary situation, according to the present invention, of securely revealing personal identifiers of two persons.

### DETAILED DESCRIPTION

The present invention will be described with respect to particular embodiments and with reference to certain drawings, but the invention is not limited thereto but only by the claims. The drawings described are only schematic and are nonlimiting. In the drawings, the size of some of the elements may be exaggerated and not drawn on scale for illustrative purposes.

Where an indefinite or definite article is used when referring to a singular noun, e.g. "a", "an", "the", this includes a plural of that noun unless something else is specifically stated.

Furthermore, the terms first, second, third and the like in the description and in the claims are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In Figure 1, a plurality of persons 100 in a contact detection and/or contact tracing setting are schematically represented, wherein each of the plurality of persons 100 is wearing or carrying a wearable or portable device (or tag) of a plurality of wearable or portable devices (or tags) 200, wherein for the sake of simplicity, Figure 1 only shows wearable or portable devices 200 for four persons 100 explicitly.

In Figure 2, a contact situation of a (specific) wearable or portable device 201 with a (specific) further wearable or portable device 202 is schematically shown. Each of the wearable or portable devices 201, 202 comprise, respectively, a wireless communication interface 220, and each of the wearable or portable devices 201, 202 comprise, respectively, a tag identifier, the wearable or portable device's tag identifier being indicated, in Figure 2, by means of reference sign 241, and the further wearable or portable device's tag identifier being indicated by means of reference sign 242. By means of the wearable or portable device's wireless communication interface 220, the wearable or portable device 201 is able, with respect to the further wearable or portable device 202, to determine repeatedly the or its distance (i.e. the distance of the wearable or portable device 201) towards the further wearable or portable device 202 and to receive the further wearable or portable device's tag identifier 242 (by means of receiving radio frequency signals broadcast by the further wearable or portable device 202 and comprising the further wearable or portable device's tag identifier 242), thereby generating and storing log data of the contact situation of the wearable or portable device 201 with the further wearable or portable device 202. Of course, analogous contact situations will typically also arise with respect to still further wearable or portable device (not individually indicated by means of references signs), i.e. the wearable or portable device 201 will typically also receive the broadcast signals of these still further wearable or portable devices, and, consequently, the log data will typically also comprise information regarding the corresponding contact situations.

Hence, by means of the wearable or portable devices 201, 202 repeatedly determining the respective distances towards each other they continuously receive the respective other wearable or portable device's tag identifier, 242, 241, thereby generating and storing the log data of especially the contact situation of the wearable or portable device 201 with the further wearable or portable device 202 (typically not only thereof but likely also information regarding additional contact situations of the wearable or portable device 201 with still further wearable or portable device, i.e. this information (i.e. the respective distance data for different points in time and the still further wearable or portable device's tag identifiers) is typically also part of the log data).

In Figure 3, an exemplary embodiment of the method according to the present invention is schematically shown by means of schematically illustrating an exemplary situation according to the present invention for securely revealing a personal identifier 541, 542 of or related to two persons, respectively, to a contact tracing entity 500 of a contact tracing system or method based on detecting relevant encounters between two persons. It is supposed that each of the plurality of persons 100 are wearing or carrying a wearable or portable device 201, 202, the wearable or portable devices 201, 202 having or being associated or assigned to, respectively, a tag identifier 241, 242, the tag identifiers 241, 242 being repeatedly broadcast by the wearable or portable devices 201, 202, respectively, using a wireless communication interface of the wearable or portable devices 201, 202, thereby generating and/or storing log data from which, respectively, encounter event related data 511 and further encounter event related data 512 are able to be derived (especially by a corresponding computing device or functionality 151, 152). The encounter event related data 511 of, or generated by the wearable or portable device 201 with respect to the further wearable or portable device 202 thus comprises both tag identifiers of both wearable or portable devices 201, 202 concerned. Likewise, the encounter event related data 512 of, or generated by the further wearable or portable device 202 (with respect to the wearable or portable device 201) also comprises both tag identifiers of both wearable or portable devices 201, 202 concerned. Furthermore, the computing device or functionality 151 is associated or related to the wearable or portable device 201, and a further computing device or functionality 152 is associated or related to the further wearable or portable device 202; these two functionalities being able to be either separated (i.e. by means of using tags as wearable or portable device, and mobile phones or other personal computing devices as computing device or functionality), or these functionalities might also be integrated in one device such as a smart phone or the like.

According to the present invention, a hash function 545 is used for securely revealing the personal identifiers 541, 542 in case that the concerned persons are both opting-in for such revealing. This is realized by means of the following steps.

In a first step, a relevant encounter 510 of the wearable or portable device 201 and the further wearable or portable device 202 is detected, independently from each other, by both the wearable or portable device 201 and the further wearable or portable device 202; this triggers the wearable or portable device 201 to generate and to transmit the corresponding encounter event related data 511 - comprising both tag identifiers 241, 242 (of the wearable or portable devices involved) as well as at least one encounter time stamp information - to the computing device or functionality 151; furthermore, this triggers the further wearable or portable device 202 to generate and to transmit the corresponding further encounter event related data 512 - comprising both tag identifiers 241, 242 (of the wearable or portable devices involved) as well as at least one further encounter time stamp information - to the further computing device or functionality 152.

In a second step, the computing device or functionality 151 generates upload data 551 and the further computing device or functionality 152 generates further upload data 552, both the computing device or functionality 151 and the further computing device or functionality 152 transmitting the respective upload data 551, 552 to the contact tracing entity 500. In case both persons do not have reasons to believe they are infected or are not informed about a diagnosis, the respective computing device or functionality 151, 152 is using the hash function 545 to generate hash values based on both wearable or portable device's tag identifiers - hereinafter also called bi-hash - in a specific manner such that among such hash values no corresponding data are able to detected. However, in case one of the persons is diagnosed, the bi-hash is also generated, by the corresponding computing device or functionality 151, 152, however in a different manner, such that - in case of a relevant encounter 510 of the wearable or portable devices 201, 202 concerned - the corresponding bi-hash values are identical, and, thus, are able to be associated to each other.
In more detail, the computing device or functionality 151, upon being informed about a diagnosis, especially a positive test result, generates a diagnosis hash value 533 by computing the hash function 545 from a diagnosis hash input information 523, comprising both tag identifiers 241, 242 in a diagnosis order, especially the wearable or portable device's tag identifier 241 first, the diagnosis hash value 533 as well as the personal identifier 541 being part of the upload data 551.
Likewise, the further computing device or functionality 152 generates a further hash value 532 by computing the hash function 545 from a hash input information 522 comprising both tag identifiers 241, 242 in a standard order, especially the wearable or portable device's tag identifier 241 first, the further hash value 532 as well as the further personal identifier 542 being part of the further upload data 552. This results in the contact tracing entity 500 being able to know, by means of both the diagnosis hash value 533 and the further hash value 532 being identical, about the relevant encounter 510 of the wearable or portable device 201 (diagnosed - or, rather the corresponding person having been diagnosed) and the further wearable or portable device 202 as well as of the personal identifiers 541, 542.

In a situation where the computing device or functionality 151 is not informed about a diagnosis - i.e. in the absence of an information about a diagnosis, especially in the absence of a positive test result - (during, prior to or after the second step), the computing device or functionality 151 generates, a hash value 531, as part of the upload data 551, by computing the hash function 545 from a hash input information 521, comprising both tag identifiers 241, 242 in the standard order as well (especially the further wearable or portable device's tag identifier 242 first), resulting in the contact tracing entity 500 being unable to know about the encounter of the wearable or portable device 201 and the further wearable or portable device 202, by means of the hash value 531 and the further hash value 532 being different from each other.

Both cases or both situations (i.e. the computing device or functionality 151 being either informed about a diagnosis (and applying the diagnosis order of hashing the tag identifiers), and not being informed about a diagnosis (hence, applying the standard order of hashing the tag identifiers)) is schematically shown on the left hand side of Figure 3, the different corresponding possibilities of generating hash values being schematically shown in the lower part of Figure 3.

According to the present invention, it is preferred that the both the upload data 551 and the further upload data 552 - in addition to the hash values 533, 532, 531, and the personal identifiers 541, 542 - furthermore also comprise at least one piece of time stamp information, respectively, related to the relevant encounter 510 of the wearable or portable device 201 and the further wearable or portable device 202. Especially, the piece of time stamp information of the upload data 551:
-- in case of the computing device or functionality 151 being informed about a diagnosis, corresponds to both the beginning and the end of the relevant encounter 510,
-- in case of the absence of an information about a diagnosis received by the computing device or functionality 151, corresponds to a point in time in between the beginning and the end of the relevant encounter 510, as detected by the wearable or portable device 201, especially corresponding to the arithmetic mean of the beginning and the end of the relevant encounter 510, and
wherein especially the piece of time stamp information of the further upload data 552 in case of the absence of an information about a diagnosis received by the further computing device or functionality 152, corresponds to a point in time in between the beginning and the end of the relevant encounter 510, as detected by the further wearable or portable device 202, especially corresponding to the arithmetic mean of the beginning and the end of the relevant encounter 510.

It is especially preferred that both the diagnosis hash input information 523, and the standard hash input information 521, 522 comprise, besides both tag identifiers 241, 242, a further information, the further information being common to both the wearable or portable device 201 and the further wearable or portable device 202 but continuously changing over time, the further information especially corresponding to the current date, or the current week of the relevant encounter 510.

Hence, according to the present invention, it is advantageously possible to realize a functionality where wearable or portable devices produce detailed contact logs (e.g. CSV files, where each line is a quadruple <My ID; Other ID; Timestamp; Distance>), especially by means of ultra-wideband, UWB, wearable devices or tags. These contact logs or log data is processed on the respective users' smartphones (or the corresponding computing device or functionality). According to the present invention, it is advantageously possible that users with a diagnosis (e.g. a positive Covid-19 diagnosis) may (voluntarily but semi-automatically) share the tag identifiers (or IDs) of the tags they used plus the times of usage. This enables a program or an app (or mobile app) of other users to compute the infection risk based on contact times and distances. However, in case such revealing of personal information is not mandatory, revealing a part of such relevant encounter events might also reveal data related to users who did not want to opt-in. According to the present invention, it is advantageously possible to provide a solution to reveal information or data (as requested) regarding "opt-in"-users (such that infected users are able to voluntarily share their identity and, additionally, healthy users who receive a risk alert can also share their identity) while avoiding to reveal information about "opt-out"-users.

According to the present invention, infected persons are able to be connected to all those with whom they had a risk contact, e.g. on a company dashboard, for helping to counter the spread of disease, e.g. Covid-19. According to the present invention, it is advantageously possible to realize a communication between two app users such that they can communicate with each other, even though the corresponding (mobile) app itself is neither personalized nor registered pseudonymously. Furthermore, it is advantageously possible to avoid revealing data of persons that did not opt-in to the data sharing or revealing mechanism. This also means that risk alerts of healthy users must be discarded if the infected user that gave rise to the alert didn't opt in.

According to the present invention, the publication of IDs by an infected user (i.e. the corresponding transmission of the upload data 551 or further upload data 552) always precedes risk alerts on the phones of healthy users (i.e. the corresponding transmission of the upload data 551 or further upload data 552) based on these published IDs. The sequence of time is important here. According to the present invention, whole encounters (i.e. log data comprising especially lists of contacts or distance detection events) are not necessarily required: the upload data 551 and the further upload data 552 furthermore also comprises at least one piece of time stamp information such as to be able to reveal - in case of an infected user - preferably both start and end times of the encounters. Furthermore according to the present invention, a risk encounter of a healthy user (Bob), which has been found using the algorithm X (i.e. the detection and generation of the relevant encounter 510 and the corresponding data generated by the corresponding wearable or portable devices 201, 202 relate to the same or similar detection algorithm employed both by the wearable or portable device 201 and the further wearable or portable device 202), can also be found in the logs (i.e. uploaded upload data 551) of the infected user (Alice) using the same or similar algorithm X, give or take a few seconds at the time interval borders (as, typically, tags (or wearable or portable devices) don't scan perfectly in sync). According to the present invention, Bob's "My ID" (i.e. the respective tag identifier 242) is or corresponds to Alice's "Other ID" during this encounter (and vice versa), and both know who's the infected person and who's at risk (or, rather, both know of themselves which health status they have), hence, these things or pieces of information are a shared secret of both persons (Alice and Bob). In case the diagnosed user or person (Alice) decides to upload her risk encounters (pertaining to those whom she put at risk, i.e. her upload data 551), using a clear name or a pseudonym (for herself, i.e. her personal identifier 541): For each of her risk encounters, her upload data 551 especially comprises the start time, the end time (i.e. the at least one piece of time stamp information), and the bi-hash (i.e. the diagnosis hash value 533), which is the hash (say, according to the SHA-256, or SHA-384, or SHA-512) of her own ID appended by the contact's ID ("Other ID"), briefly, bi-hash = hash (Own ID + Other ID), i.e. the diagnosed user or person (Alice) applies the hash algorithm or hash function 545 to both tag identifiers concerned 241, 242 according to the diagnosis order (e.g., own tag identifier first). Now, if the undiagnosed user or person (Bob) also selected opt-in, his clear name or pseudonym (i.e. his personal identifier 542) is uploaded (as part of the upload data 552), and for each of his risk encounters (which may or may not result from contacts with the infected user (Alice)), he uploads the bi-hash (for him, hash(Other ID + Own ID), i.e. the undiagnosed user or person (Bob) applies the hash algorithm or hash function 545 to both tag identifiers concerned 241, 242 according to the standard order (e.g., the other ID, or tag identifier, first), since especially the ID of the infected person comes first, and, hence, Bob puts his ID second) and one timestamp in the middle of the encounter. Now, if the bi-hash (i.e. the further hash value 532) of one of Bob's upload items matches the bi-hash of a stored record, and if the midpoint (in time, i.e. the corresponding time stamp information) provided by Bob lies within the encounter start and end times (of the log data provided by Alice), Bob's name (or pseudonym, i.e. his personal identifier 542) is stored in that record. If the bi-hash of one of Bob's upload items can't be found, the infected user behind the encounter didn't opt in - as that would have, especially, happened earlier. If a sufficiently strong hash function is used, it is practically impossible to retrieve the IDs (i.e. the tag identifiers) from only knowing the hash values 531, 532, 533; certainly, the security depends on the length of the tag identifiers 241, 242 (128 bits or more is preferred according to the present invention) and on randomness of the tag identifiers 241, 242. It is especially preferred according to the present invention that the upload data of a diagnosed user or person (Alice in the example) is secured using a TAN verification process, especially the TAN verification process that is also used for sharing her, i.e. the process that is used to upload the anonymous information relating to Alice's infection or diagnosis.

According to the present invention and regarding the example mentioned, Alice does not expose anything that could be realistically traced back to users without opt-in. Alice preferably never knows the identity of those she puts at risk. The bi-hash = hash function (Infected ID + Other ID)
can be computed by both Alice and Bob, but by no one else, and the bi-hash does not reveal the underlying tag identifiers 241, 242, since the hash is a one-way function. The web service, i.e. the contact tracing entity 500, validates for each of Bob's contacts if his hash value (bi-hash) is identical with an especially already uploaded diagnosis hash value 533 present on the contact tracing entity 500. If and only if so, Bob's name goes to that record; Bob preferably never knows the identity of those who put him at risk - only the contact tracing entity 500, e.g. a human resources department of an enterprise, knows about the risk encounters only if the infected user opted in; likewise, the contact tracing entity 500 knows the identity of the at-risk users only if they also opted in, hence, the risk graph is only known to the contact tracing entity 500, not to the users.

## Claims

1. Method for securely revealing a personal identifier (541, 542) of or related to two persons, respectively, to a contact tracing entity (500) of a contact tracing system or method based on detecting relevant encounters between two persons, wherein each of the plurality of persons (100) are wearing or carrying a wearable or portable device (201, 202), the wearable or portable devices (201, 202) having or being associated or assigned to, respectively, a tag identifier (241, 242), the tag identifiers (241, 242) being repeatedly broadcast by the wearable or portable devices (201, 202), respectively, using a wireless communication interface of the wearable or portable devices (201, 202), thereby generating and/or storing log data from which, respectively, encounter event related data (511) and further encounter event related data (512) are able to be derived, comprising, respectively, both tag identifiers (241, 242), a computing device (151) being associated or related to the wearable or portable device (201), as well as a further computing device (152) being associated or related to the further wearable or portable device (202),
wherein a hash function (545) is used for securely revealing the personal identifiers (541, 542) in case that the concerned persons are both opting-in for such revealing, the method comprises the following steps:
-- in a first step, a relevant encounter (510) of the wearable or portable device (201) and the further wearable or portable device (202) is detected, independently from each other, by the wearable or portable device (201) and the further wearable or portable device (202), thereby generating and transmitting
-- the corresponding encounter event related data (511), comprising both tag identifiers (241, 242) as well as at least one encounter time stamp information, to the computing device (151), and
-- the corresponding further encounter event related data (512), comprising both tag identifiers (241, 242) as well as at least one further encounter time stamp information, to the further computing device (152),
-- in a second step, the computing device (151) generates upload data (551) and the further computing device (152) generates further upload data (552), both the computing device (151) and the further computing device (152) transmitting the respective upload data (551, 552) to the contact tracing entity (500), wherein
-- the computing device (151), upon being informed about a diagnosis, especially a positive test result, generates a diagnosis hash value (533) by computing the hash function (545) from a diagnosis hash input information (523), comprising both tag identifiers (241, 242) in a diagnosis order, especially the wearable or portable device's tag identifier (241) first, the diagnosis hash value (533) as well as the personal identifier (541) being part of the upload data (551), and
-- the further computing device (152) generates a further hash value (532) by computing the hash function (545) from a hash input information (522) comprising both tag identifiers (241, 242) in a standard order, especially the wearable or portable device's tag identifier (241) first, the further hash value (532) as well as the further personal identifier (542) being part of the further upload data (552),
resulting in the contact tracing entity (500) being able to know, by means of both the diagnosis hash value (533) and the further hash value (532) being identical, about the relevant encounter (510) of the wearable or portable device (201) and the further wearable or portable device (202) as well as of the personal identifiers (541, 542).

2. Method according to claim 1, wherein, during, prior to or after the second step, the computing device (151) generates, in the absence of an information about a diagnosis, especially a positive test result, a hash value (531), as part of the upload data (551), by computing the hash function (545) from a hash input information (521), comprising both tag identifiers (241, 242) in the standard order, especially the further wearable or portable device's tag identifier (242) first, resulting in the contact tracing entity (500) being unable to know about the encounter of the wearable or portable device (201) and the further wearable or portable device (202), by means of the hash value (531) and the further hash value (532) being different from each other.

3. Method according to one of the preceding claims, wherein both the upload data (551) and the further upload data (552) furthermore also comprises at least one piece of time stamp information, respectively, related to the relevant encounter (510) of the wearable or portable device (201) and the further wearable or portable device (202),
wherein especially the piece of time stamp information of the upload data (551)
-- in case of the computing device (151) being informed about a diagnosis, corresponds to both the beginning and the end of the relevant encounter (510),
-- in case of the absence of an information about a diagnosis received by the computing device (151), corresponds to a point in time in between the beginning and the end of the relevant encounter (510), as detected by the wearable or portable device (201), especially corresponding to the arithmetic mean of the beginning and the end of the relevant encounter (510), and
wherein especially the piece of time stamp information of the further upload data (552) in case of the absence of an information about a diagnosis received by the further computing device (152), corresponds to a point in time in between the beginning and the end of the relevant encounter (510), as detected by the further wearable or portable device (202), especially corresponding to the arithmetic mean of the beginning and the end of the relevant encounter (510).

4. Method according to one of the preceding claims, wherein - in case that the contact tracing entity (500) is able to know, by means of both the diagnosis hash value (533) and the further hash value (532) being identical, about the relevant encounter (510) of the wearable or portable device (201) and the further wearable or portable device (202) - the contact tracing entity (500) is also able to know that the at least one piece of time stamp information of both the upload data (551) and the further upload data (552) are corresponding to each other, especially by means of the piece of time stamp information of the further upload data (552) indicating a point in time in between the points in time indicated by the piece of time stamp information of the upload data (551).

5. Method according to one of the preceding claims, wherein both the diagnosis hash input information (523), and the standard hash input information (521, 522) comprise, besides both tag identifiers (241, 242), a further information, the further information being common to both the wearable or portable device (201) and the further wearable or portable device (202) but continuously changing over time, the further information especially corresponding to the current date, or the current week of the relevant encounter (510).

6. Method according to one of the preceding claims, wherein the personal identifier is or corresponds to an information relating to the identity of the respective person, especially the name or a further identity information, or to a pseudonym and/or wherein at least a part of the plurality of persons (100) is opting-in for revealing the respective personal identifier.

7. Method according to one of the preceding claims, wherein, in a contact situation of the wearable or portable device (201) with the further wearable or portable device (202), the wearable or portable device (201) is able to repeatedly determine the distance, at different points in time, towards the further wearable or portable device (202), and vice versa, and to receive the further wearable or portable device's tag identifier (242) by the wearable or portable device (201) and the wearable or portable device's tag identifier (241) by the further wearable or portable device (202), thereby especially generating and storing log data.

8. System for securely revealing a personal identifier (541, 542) of or related to two persons, respectively, to a contact tracing entity (500) of a contact tracing system or method based on detecting relevant encounters between two persons,
the system comprising, besides a plurality of wearable or portable devices (200), the contact tracing entity (500), and a plurality of computing devices (151, 152), wherein each of the plurality of persons (100) are wearing or carrying a wearable or portable device (201, 202), the wearable or portable devices (201, 202) having or being associated or assigned to, respectively, a tag identifier (241, 242), the tag identifiers (241, 242) being repeatedly broadcast by the wearable or portable devices (201, 202), respectively, using a wireless communication interface of the wearable or portable devices (201, 202), thereby generating and/or storing log data from which, respectively, encounter event related data (511) and further encounter event related data (512) are able to be derived, comprising, respectively, both tag identifiers (241, 242), a computing device (151) being associated or related to the wearable or portable device (201), as well as a further computing device (152) being associated or related to the further wearable or portable device (202),
wherein a hash function (545) is used for securely revealing the personal identifiers (541, 542) in case that the concerned persons are both opting-in for such revealing, the system being configured such that:
-- a relevant encounter (510) of the wearable or portable device (201) and the further wearable or portable device (202) is detected, independently from each other, by the wearable or portable device (201) and the further wearable or portable device (202), thereby generating and transmitting
-- the corresponding encounter event related data (511), comprising both tag identifiers (241, 242) as well as at least one encounter time stamp information, to the computing device (151), and
-- the corresponding further encounter event related data (512), comprising both tag identifiers (241, 242) as well as at least one further encounter time stamp information, to the further computing device (152),
-- the computing device (151) generates upload data (551) and the further computing device (152) generates further upload data (552), both the computing device (151) and the further computing device (152) transmitting the respective upload data (551, 552) to the contact tracing entity (500), wherein
-- the computing device (151), upon being informed about a diagnosis, especially a positive test result, generates a diagnosis hash value (533) by computing the hash function (545) from a diagnosis hash input information (523), comprising both tag identifiers (241, 242) in a diagnosis order, especially the wearable or portable device's tag identifier (241) first, the diagnosis hash value (533) as well as the personal identifier (541) being part of the upload data (551), and
-- the further computing device (152) generates a further hash value (532) by computing the hash function (545) from a hash input information (522) comprising both tag identifiers (241, 242) in a standard order, especially the wearable or portable device's tag identifier (241) first, the further hash value (532) as well as the further personal identifier (542) being part of the further upload data (552),
resulting in the contact tracing entity (500) being able to know, by means of both the diagnosis hash value (533) and the further hash value (532) being identical, about the relevant encounter (510) of the wearable or portable device (201) and the further wearable or portable device (202) as well as of the personal identifiers (541, 542).

9. Computing devices (151, 152) and wearable or portable devices (200) for securely revealing a personal identifier (541, 542) of or related to two persons, respectively, to a contact tracing entity (500) of a contact tracing system or method based on detecting relevant encounters between two persons, wherein each of the plurality of persons (100) are wearing or carrying a wearable or portable device (201, 202), the wearable or portable devices (201, 202) having or being associated or assigned to, respectively, a tag identifier (241, 242), the tag identifiers (241, 242) being repeatedly broadcast by the wearable or portable devices (201, 202), respectively, using a wireless communication interface of the wearable or portable devices (201, 202), thereby generating and/or storing log data from which, respectively, encounter event related data (511) and further encounter event related data (512) are able to be derived, comprising, respectively, both tag identifiers (241, 242), a computing device (151) being associated or related to the wearable or portable device (201), as well as a further computing device (152) being associated or related to the further wearable or portable device (202),
wherein a hash function (545) is used for securely revealing the personal identifiers (541, 542) in case that the concerned persons are both opting-in for such revealing, the computing device and the wearable or portable devices being configured such that:
-- a relevant encounter (510) of the wearable or portable device (201) and the further wearable or portable device (202) is detected, independently from each other, by the wearable or portable device (201) and the further wearable or portable device (202), thereby generating and transmitting
-- the corresponding encounter event related data (511), comprising both tag identifiers (241, 242) as well as at least one encounter time stamp information, to the computing device (151), and
-- the corresponding further encounter event related data (512), comprising both tag identifiers (241, 242) as well as at least one further encounter time stamp information, to the further computing device (152),
-- the computing device (151) generates upload data (551) and the further computing device (152) generates further upload data (552), both the computing device (151) and the further computing device (152) transmitting the respective upload data (551, 552) to the contact tracing entity (500), wherein
-- the computing device (151), upon being informed about a diagnosis, especially a positive test result, generates a diagnosis hash value (533) by computing the hash function (545) from a diagnosis hash input information (523), comprising both tag identifiers (241, 242) in a diagnosis order, especially the wearable or portable device's tag identifier (241) first, the diagnosis hash value (533) as well as the personal identifier (541) being part of the upload data (551), and
-- the further computing device (152) generates a further hash value (532) by computing the hash function (545) from a hash input information (522) comprising both tag identifiers (241, 242) in a standard order, especially the wearable or portable device's tag identifier (241) first, the further hash value (532) as well as the further personal identifier (542) being part of the further upload data (552),
resulting in the contact tracing entity (500) being able to know, by means of both the diagnosis hash value (533) and the further hash value (532) being identical, about the relevant encounter (510) of the wearable or portable device (201) and the further wearable or portable device (202) as well as of the personal identifiers (541, 542).

10. Program comprising a computer readable program code, which, when executed on a computer and/or on a computing device (151) and/or on a contact tracing entity (500) and/or on a wearable or portable device (201), or in part on a computing device (151) and/or in part on a contact tracing entity (500) and/or on a wearable or portable device (201), causes the computer and/or the computing device (151) and/or the contact tracing entity (500) and/or the wearable or portable device (201) to perform a method according one of claims 1 to 7.

11. Computer-readable medium comprising instructions which when executed on a computer and/or on a computing device (151) and/or on a contact tracing entity (500) and/or on a wearable or portable device (201), or in part on a computing device (151) and/or in part on a contact tracing entity (500) and/or on a wearable or portable device (201), causes the computer and/or the computing device (151) and/or the contact tracing entity (500) and/or the wearable or portable device (201) to perform a method according one of claims 1 to 7.

## Patentansprüche

1. Verfahren zum sicheren Offenlegen eines persönlichen Identifikators (541, 542) von oder in Bezug auf jeweils zwei Personen gegenüber einer Kontaktverfolgungseinrichtung (500) eines Kontaktverfolgungssystems oder - verfahrens auf der Grundlage der Erfassung relevanter Begegnungen zwischen zwei Personen, wobei jede der mehreren Personen (100) ein tragbares Gerät (201, 202) trägt oder bei sich trägt, wobei die tragbaren Geräte (201, 202) (201, 202) jeweils einen Tag-Identifikator (241, 242) aufweisen oder diesem zugeordnet sind, wobei die Tag-Identifikatoren (241, 242) wiederholt von den tragbaren Geräten (201, 202) unter Verwendung einer drahtlosen Kommunikationsschnittstelle der tragbaren Geräte (201, 202) gesendet werden, wodurch Log-Daten erzeugt und/oder gespeichert werden, aus denen jeweils Begegnungsereignis-bezogene Daten (511) und weitere Begegnungsereignis-bezogene Daten (512) abgeleitet werden können, die jeweils die beiden Tag-Identifikatoren (241, 242) umfassen, wobei eine Rechnereinrichtung (151) dem tragbaren Gerät (201) zugeordnet oder mit diesem verbunden ist sowie eine weitere Rechnereinrichtung (152) dem weiteren tragbaren Gerät (202) zugeordnet oder mit diesem verbunden ist, wobei eine Hashfunktion (545) verwendet wird, um die persönlichen Identifikatoren (541, 542) sicher offenzulegen, falls die betroffenen Personen beide einer solchen Offenlegung zustimmen, wobei das Verfahren die folgenden Schritte umfasst:
-- in einem ersten Schritt wird eine relevante Begegnung (510) des tragbaren Geräts (201) und des weiteren tragbaren Geräts (202) unabhängig voneinander durch das tragbare Gerät (201) und das weitere tragbare Gerät (202) erkannt, wobei
-- die entsprechenden Begegnungsereignis-bezogenen Daten (511), die sowohl die Tag-Identifikatoren (241, 242) als auch mindestens eine Begegnungs-Zeitstempel-Information umfassen, erzeugt und an die Rechnereinrichtung (151) übertragen werden, und
-- die entsprechenden weiteren Begegnungsereignis-bezogenen Daten (512), die sowohl Tag-Identifikatoren (241, 242) als auch mindestens eine weitere Begegnungs-Zeitstempel-Information umfassen, erzeugt und an die weitere Rechnereinrichtung (152) übertragen werden,
-- in einem zweiten Schritt erzeugt die Rechnereinrichtung (151) Upload-Daten (551) und die weitere Rechnereinrichtung (152) erzeugt weitere Upload-Daten (552), wobei sowohl die Rechnereinrichtung (151) als auch die weitere Rechnereinrichtung (152) die jeweiligen Upload-Daten (551, 552) an die Kontaktverfolgungseinrichtung (500) übermitteln, wobei
-- die Rechnereinrichtung (151) bei Information über eine Diagnose, insbesondere ein positives Testergebnis, einen Diagnose-Hashwert (533) durch Berechnung der Hashfunktion (545) aus einer Diagnose-Hash-Eingabeinformation (523) berechnet, die sowohl Tag-Identifikatoren (241, 242) in einer Diagnose-Reihenfolge, insbesondere zuerst den Tag-Identifikator (241) des tragbaren Geräts, umfasst, wobei der Diagnose-Hashwert (533) sowie der persönliche Identifikator (541) Teil der Upload-Daten (551) sind, und
-- die weitere Rechnereinrichtung (152) erzeugt einen weiteren Hashwert (532) durch Berechnen der Hashfunktion (545) aus einer Hash-Eingabeinformation (522), die beide Tag-Identifikatoren (241, 242) in einer Standardreihenfolge umfasst, insbesondere den Tag-Identifikator (241) des tragbaren Geräts zuerst, wobei der weitere Hashwert (532) sowie der weitere persönliche Identifikator (542) Teil der weiteren Upload-Daten (552) ist,
was dazu führt, dass die Kontaktverfolgungseinrichtung (500) basierend darauf, dass sowohl der Diagnose-Hashwert (533) als auch der weitere Hashwert (532) identisch sind, Kenntnis über die relevante Begegnung (510) des tragbaren oder mobilen Geräts (201) und des weiteren tragbaren Geräts (202) sowie der persönlichen Identifikatoren (541, 542) haben kann.

2. Verfahren nach Anspruch 1, wobei die Rechnereinrichtung (151) während, vor oder nach dem zweiten Schritt in Abwesenheit einer Information über eine Diagnose, insbesondere ein positives Testergebnis, einen Hashwert (531) als Teil der Upload-Daten (551) erzeugt, indem sie die Hashfunktion (545) aus einer Hash-Eingabeinformation (521) berechnet, die beide Tag-Identifikatoren (241, 242) in der Standardreihenfolge umfasst, insbesondere der Tag-Identifikator (241) des weiteren tragbaren oder portablen Geräts zuerst, so dass die Kontaktverfolgungseinrichtung (500) nicht in der Lage ist, von der Begegnung des tragbaren Geräts (201) und dem weiteren tragbaren Gerät (202) zu wissen, indem der Hashwert (531) und der weitere Hashwert (532) voneinander verschieden sind.

3. Verfahren nach einem der vorstehenden Ansprüche, wobei sowohl die Upload-Daten (551) als auch die weiteren Upload-Daten (552) darüber hinaus jeweils mindestens eine Zeitstempel-Information umfassen, die sich auf die relevante Begegnung (510) des tragbaren Geräts (201) und des weiteren tragbaren Geräts (202) bezieht,
wobei insbesondere die Zeitstempel-Information der Upload-Daten (551)
-- im Fall, dass die Rechnereinrichtung (151) über eine Diagnose informiert ist, sowohl dem Beginn als auch dem Ende der relevanten Begegnung (510) entspricht,
-- im Fall, dass keine Information über eine Diagnose bei der Rechnereinrichtung (151) eingegangen ist, einem Zeitpunkt zwischen dem Beginn und dem Ende der relevanten Begegnung (510), wie von dem tragbaren Gerät (201) erfasst, entspricht, insbesondere dem arithmetischen Mittelwert des Beginns und des Endes der relevanten Begegnung (510), und
wobei insbesondere der Teil der Zeitstempel-Information der weiteren Upload-Daten (552) im Falle des Fehlens einer Information über eine Diagnose, die von der weiteren Rechnereinrichtung (152) empfangen wurde, einem Zeitpunkt zwischen dem Beginn und dem Ende der relevanten Begegnung (510), wie von der weiteren tragbaren oder mobilen Einrichtung (202) erfasst, entspricht, insbesondere entsprechend dem arithmetischen Mittelwert des Beginns und des Endes der relevanten Begegnung (510).

4. Verfahren nach einem der vorstehenden Ansprüche, wobei - für den Fall, dass die Kontaktverfolgungseinrichtung (500) anhand der Identität des Diagnose-Hashwerts (533) und des weiteren Hashwerts (532) von der relevanten Begegnung (510) des tragbaren Geräts (201) und des weiteren tragbaren Geräts (202) Kenntnis erlangt - die Kontaktverfolgungseinrichtung (500) auch erkennen kann, dass die mindestens eine Zeitstempel-Information sowohl der Upload-Daten (551) als auch der weiteren Upload-Daten (552) einander entsprechen, insbesondere dadurch, dass die Zeitstempel-Information der weiteren Upload-Daten (552) einen Zeitpunkt zwischen den durch die Zeitstempel-Information der Upload-Daten (551) angegebenen Zeitpunkten angibt.

5. Verfahren nach einem der vorstehenden Ansprüche, wobei sowohl die Diagnose-Hash-Eingabeinformation (523) als auch die Standard-Hash-Eingabeinformation (521, 522) neben beiden Tag-Identifikatoren (241, 242) eine weitere Information umfasst, wobei die weitere Information dem tragbaren Gerät (201) und dem weiteren tragbaren Gerät (202) gemeinsam ist, wobei die weitere Information insbesondere dem aktuellen Datum oder der aktuellen Woche der relevanten Begegnung (510) entspricht.

6. Verfahren nach einem der vorstehenden Ansprüche, wobei der persönliche Identifikator eine Information ist oder einer Information entspricht, die sich auf die Identität der jeweiligen Person, insbesondere den Namen oder eine weitere Identitätsinformation, oder auf ein Pseudonym bezieht, und/oder wobei zumindest ein Teil der mehreren Personen (100) sich für die Offenlegung des jeweiligen persönlichen Identifikators entscheidet.

7. Verfahren nach einem der vorstehenden Ansprüche, wobei in einer Kontaktsituation des tragbaren oder mitführbaren Geräts (201) mit dem weiteren tragbaren oder mitführbaren Gerät (202) das tragbare oder mitführbare Gerät (201) in der Lage ist, wiederholt den Abstand zu dem weiteren tragbaren oder mitführbaren Gerät (202) zu unterschiedlichen Zeitpunkten zu bestimmen und umgekehrt, und zum Empfangen des Tag-Identifikators (242) des weiteren tragbaren Geräts durch das tragbare Gerät (201) und des Tag-Identifikators (241) des weiteren tragbaren Geräts durch das weitere tragbare Gerät (202), wobei insbesondere Log-Daten erzeugt und gespeichert werden.

8. System zum sicheren Offenlegen eines persönlichen Identifikators (541, 542) von oder in Bezug auf jeweils zwei Personen gegenüber einer Kontaktverfolgungseinrichtung (500) eines Kontaktverfolgungssystems oder - verfahrens auf der Grundlage der Erfassung relevanter Begegnungen zwischen zwei Personen, wobei das System neben einer Mehrzahl von tragbaren Geräten (200) die Kontaktverfolgungseinrichtung (500) und eine Mehrzahl von Rechnereinrichtungen (151, 152) aufweist,
wobei jede der Mehrzahl von Personen (100) ein tragbares Gerät (201, 202) trägt oder bei sich trägt, wobei die tragbaren Geräte (201, 202) einen Tag-Identifikator (241, 242) aufweisen oder diesem zugeordnet sind bzw. diesem zugewiesen sind, wobei die Tag-Identifikatoren (241, 242) wiederholt von den tragbaren Geräten (201, 202) unter Verwendung einer drahtlosen Kommunikationsschnittstelle der tragbaren Geräte (201, 202) gesendet werden, wodurch Log-Daten erzeugt und/oder gespeichert werden, aus denen jeweils Begegnungsereignis-bezogene Daten (511) und weitere Begegnungsereignis-bezogene Daten (512) abgeleitet werden können, die jeweils die beiden Tag-Identifikatoren (241, 242) umfassen, wobei eine Rechnereinrichtung (151) dem tragbaren Gerät (201) zugeordnet oder mit diesem verbunden ist, sowie eine weitere Rechnereinrichtung (152) dem weiteren tragbaren Gerät (202) zugeordnet oder mit diesem verbunden ist, wobei eine Hashfunktion (545) verwendet wird, um die persönlichen Identifikatoren (541, 542) sicher offenzulegen, falls die betroffenen Personen beide einer solchen Offenlegung zustimmen, wobei das System so konfiguriert ist, dass:
-- eine relevante Begegnung (510) des tragbaren Geräts (201) und des weiteren tragbaren Geräts (202) wird unabhängig voneinander durch das tragbare Gerät (201) und das weitere tragbare Gerät (202) erkannt, wobei
-- die entsprechenden Begegnungsereignis-bezogenen Daten (511), die sowohl die Tag-Identifikatoren (241, 242) als auch mindestens eine Begegnungs-Zeitstempel-Information umfassen, erzeugt und an die Rechnereinrichtung (151) übertragen werden, und
-- die entsprechenden weiteren Begegnungsereignis-bezogenen Daten (512), die sowohl Tag-Identifikatoren (241, 242) als auch mindestens eine weitere Begegnungs-Zeitstempel-Information umfassen, erzeugt und an die weitere Rechnereinrichtung (152) übertragen werden,
-- die Rechnereinrichtung (151) erzeugt Upload-Daten (551) und die weitere Rechnereinrichtung (152) erzeugt weitere Upload-Daten (552), wobei sowohl die Rechnereinrichtung (151) als auch die weitere Rechnereinrichtung (152) die jeweiligen Upload-Daten (551, 552) an die Kontaktverfolgungseinrichtung (500) übermitteln, wobei
-- die Rechnereinrichtung (151) bei Information über eine Diagnose, insbesondere ein positives Testergebnis, einen Diagnose-Hashwert (533) durch Berechnung der Hashfunktion (545) aus einer Diagnose-Hash-Eingabeinformation (523) berechnet, die sowohl Tag-Identifikatoren (241, 242) in einer Diagnose-Reihenfolge, insbesondere zuerst den Tag-Identifikator (241) des tragbaren Geräts, umfasst, wobei der Diagnose-Hashwert (533) sowie der persönliche Identifikator (541) Teil der Upload-Daten (551) sind, und
-- die weitere Rechnereinrichtung (152) erzeugt einen weiteren Hashwert (532) durch Berechnen der Hashfunktion (545) aus einer Hash-Eingabeinformation (522), die beide Tag-Identifikatoren (241, 242) in einer Standardreihenfolge umfasst, insbesondere den Tag-Identifikator (241) des tragbaren Geräts zuerst, wobei der weitere Hashwert (532) sowie der weitere persönliche Identifikator (542) Teil der weiteren Upload-Daten (552) ist,
was dazu führt, dass die Kontaktverfolgungseinrichtung (500) basierend darauf, dass sowohl der Diagnose-Hashwert (533) als auch der weitere Hashwert (532) identisch sind, Kenntnis über die relevante Begegnung (510) des tragbaren oder mobilen Geräts (201) und des weiteren tragbaren Geräts (202) sowie der persönlichen Identifikatoren (541, 542) haben kann.

9. Rechnereinrichtungen (151, 152) und tragbare Geräte (200) zum sicheren Offenlegen eines persönlichen Identifikators (541, 542) von oder in Bezug auf jeweils zwei Personen gegenüber einer Kontaktverfolgungseinrichtung (500) eines Kontaktverfolgungssystems oder -verfahrens auf der Grundlage der Erfassung relevanter Begegnungen zwischen zwei Personen, wobei jede der Mehrzahl von Personen (100) ein tragbares Gerät (201, 202) trägt oder bei sich trägt, wobei die tragbaren Geräte (201, 202) einen Tag-Identifikator (241, 242) aufweisen oder diesem zugeordnet sind bzw. diesem zugewiesen sind, wobei die Tag-Identifikatoren (241, 242) wiederholt von den tragbaren Geräten (201, 202) unter Verwendung einer drahtlosen Kommunikationsschnittstelle der tragbaren Geräte (201, 202) gesendet werden, wodurch Log-Daten erzeugt und/oder gespeichert werden, aus denen jeweils Begegnungsereignis-bezogene Daten (511) und weitere Begegnungsereignis-bezogene Daten (512) abgeleitet werden können, die jeweils die beiden Tag-Identifikatoren (241, 242) umfassen, wobei eine Rechnereinrichtung (151) dem tragbaren Gerät (201) zugeordnet oder mit diesem verbunden ist, sowie eine weitere Rechnereinrichtung (152) dem weiteren tragbaren Gerät (202) zugeordnet oder mit diesem verbunden ist, wobei eine Hashfunktion (545) verwendet wird, um die persönlichen Identifikatoren (541, 542) sicher offenzulegen, falls die betroffenen Personen beide einer solchen Offenlegung zustimmen, wobei die Rechnereinrichtung und die tragbaren Geräte so konfiguriert sind, dass:
-- eine relevante Begegnung (510) des tragbaren Geräts (201) und des weiteren tragbaren Geräts (202) wird unabhängig voneinander durch das tragbare Gerät (201) und das weitere tragbare Gerät (202) erkannt, wobei
-- die entsprechenden Begegnungsereignis-bezogenen Daten (511), die sowohl die Tag-Identifikatoren (241, 242) als auch mindestens eine Begegnungs-Zeitstempel-Information umfassen, erzeugt und an die Rechnereinrichtung (151) übertragen werden, und
-- die entsprechenden weiteren Begegnungsereignis-bezogenen Daten (512), die sowohl Tag-Identifikatoren (241, 242) als auch mindestens eine weitere Begegnungs-Zeitstempel-Information umfassen, erzeugt und an die weitere Rechnereinrichtung (152) übertragen werden,
-- die Rechnereinrichtung (151) erzeugt Upload-Daten (551) und die weitere Rechnereinrichtung (152) erzeugt weitere Upload-Daten (552), wobei sowohl die Rechnereinrichtung (151) als auch die weitere Rechnereinrichtung (152) die jeweiligen Upload-Daten (551, 552) an die Kontaktverfolgungseinrichtung (500) übermitteln, wobei
-- die Rechnereinrichtung (151) bei Information über eine Diagnose, insbesondere ein positives Testergebnis, einen Diagnose-Hashwert (533) durch Berechnung der Hashfunktion (545) aus einer Diagnose-Hash-Eingabeinformation (523) berechnet, die sowohl Tag-Identifikatoren (241, 242) in einer Diagnose-Reihenfolge, insbesondere zuerst den Tag-Identifikator (241) des tragbaren Geräts, umfasst, wobei der Diagnose-Hashwert (533) sowie der persönliche Identifikator (541) Teil der Upload-Daten (551) sind, und
-- die weitere Rechnereinrichtung (152) erzeugt einen weiteren Hashwert (532) durch Berechnen der Hashfunktion (545) aus einer Hash-Eingabeinformation (522), die beide Tag-Identifikatoren (241, 242) in einer Standardreihenfolge umfasst, insbesondere den Tag-Identifikator (241) des tragbaren Geräts zuerst, wobei der weitere Hashwert (532) sowie der weitere persönliche Identifikator (542) Teil der weiteren Upload-Daten (552) ist,
was dazu führt, dass die Kontaktverfolgungseinrichtung (500) basierend darauf, dass sowohl der Diagnose-Hashwert (533) als auch der weitere Hashwert (532) identisch sind, Kenntnis über die relevante Begegnung (510) des tragbaren oder mobilen Geräts (201) und des weiteren tragbaren Geräts (202) sowie der persönlichen Identifikatoren (541, 542) haben kann.

10. Programm, das einen computerlesbaren Programmcode umfasst, der, wenn er auf einem Computer und/oder auf einer Rechnereinrichtung (151) und/oder auf einer Kontaktverfolgungseinrichtung (500) und/oder auf einem tragbaren Gerät (201) oder teilweise auf einer Rechnereinrichtung (151) und/oder teilweise auf einer Kontaktverfolgungseinrichtung (500) und/oder auf einem tragbaren Gerät (201) ausgeführt wird (201) ausgeführt wird, bewirkt, dass der Computer und/oder die Rechnereinrichtung (151) und/oder die Kontaktverfolgungseinrichtung (500) und/oder das tragbare Gerät (201) ein Verfahren gemäß einem der Ansprüche 1 bis 7 ausführt.

11. Computerlesbares Medium, das Anweisungen umfasst, die, wenn sie auf einem Computer und/oder auf einer Rechnereinrichtung (151) und/oder auf einer Kontaktverfolgungseinrichtung (500) und/oder auf einem tragbaren Gerät (201) oder teilweise auf einer Rechnereinrichtung (151) und/oder teilweise auf einer Kontaktverfolgungseinrichtung (500) und/oder auf einem tragbaren Gerät (201) ausgeführt werden (201) ausgeführt werden, den Computer und/oder die Rechnereinrichtung (151) und/oder die Kontaktverfolgungseinrichtung (500) und/oder das tragbare Gerät (201) veranlassen, ein Verfahren gemäß einem der Ansprüche 1 bis 7 auszuführen.

## Revendications

1. Procédé pour révéler de manière sécurisée un identifiant personnel (541, 542) de ou lié à deux personnes, respectivement, à une entité de recherche de contact (500) d'un système ou procédé de recherche de contact basé sur la détection de rencontres pertinentes entre deux personnes, dans lequel chacune de la pluralité de personnes (100) porte ou transporte un dispositif portable (201, 202), les dispositifs portables (201, 202) ayant ou étant associés ou attribués à, respectivement, un identifiant de balise (241, 242), les identifiants de balise (241, 242) étant diffusés de manière répétée par les dispositifs portables (201, 202), respectivement, à l'aide d'une interface de communication sans fil des dispositifs portables (201, 202), générant et/ou stockant ainsi des données de journal à partir desquelles, respectivement, des données relatives à un événement de rencontre (511) et d'autres données relatives à un événement de rencontre (512) peuvent être dérivées, comprenant, respectivement, les deux identifiants de balise (241, 242),
un dispositif informatique (151) étant associé ou lié au dispositif portable (201), ainsi qu'un autre dispositif informatique (152) associé ou lié à l'autre dispositif portable (202), dans lequel une fonction hachage (545) est utilisée pour révéler de manière sécurisée les identifiants personnels (541, 542) dans le cas où les personnes concernées optent toutes les deux pour une telle révélation, le procédé comprend les étapes suivantes :
-- dans une première étape, une rencontre pertinente (510) du dispositif portable (201) et du dispositif portable supplémentaire (202) est détectée, indépendamment l'une de l'autre, par le dispositif portable (201) et le dispositif portable supplémentaire (202), générant et transmettant ainsi
-- les données relatives à l'événement de rencontre correspondantes (511), comprenant à la fois les identifiants de balise (241, 242) et au moins une information d'horodatage de rencontre, vers le dispositif informatique (151), et
-- les autres données relatives à l'événement de rencontre correspondantes (512), comprenant à la fois les identifiants de balise (241, 242) et au moins une autre information d'horodatage de rencontre, vers le dispositif informatique supplémentaire (152),
-- dans une deuxième étape, le dispositif informatique (151) génère des données de téléchargement (551) et l'autre dispositif informatique (152) génère d'autres données de téléchargement (552), le dispositif informatique (151) et l'autre dispositif informatique (152) transmettant les données de téléchargement respectives (551, 552) à l'entité de recherche de contact (500), dans lequel
-- le dispositif informatique (151), lorsqu'il est informé d'un diagnostic, en particulier d'un résultat de test positif, génère une valeur hachage de diagnostic (533) en calculant la fonction hachage (545) à partir d'une information hachage d'entrée de diagnostic (523), comprenant les deux identifiants de balise (241, 242) dans un ordre de diagnostic, en particulier l'identifiant de balise (241) du dispositif portable en premier, la valeur hachage de diagnostic (533) ainsi que l'identifiant personnel (541) faisant partie des données de téléchargement (551), et
-- le dispositif informatique supplémentaire (152) génère une valeur hachage supplémentaire (532) en calculant la fonction hachage (545) à partir d'une information hachage d'entrée (522) comprenant les deux identifiants de balise (241, 242) dans un ordre standard, en particulier l'identifiant de balise (241) du dispositif portable en premier, la valeur hachage supplémentaire (532) ainsi que l'identifiant personnel supplémentaire (542) faisant partie des données de téléchargement supplémentaires (552), ce qui permet à l'entité de recherche de contact (500) d'être en mesure de connaître, grâce à la valeur hachage de diagnostic (533) ainsi que la valeur hachage supplémentaire (532) étant identique, concernant la rencontre pertinente (510) du dispositif portable (201) et de l'autre dispositif portable (202) ainsi que les identifiants personnels (541, 542).

2. Procédé selon la revendication 1, dans lequel, pendant, avant ou après la deuxième étape, le dispositif informatique (151) génère, en l'absence d'une information sur un diagnostic, en particulier un résultat de test positif, une valeur hachage (531), en tant que partie des données de téléchargement (551), en calculant la fonction hachage (545) à partir d'une information hachage d'entrée (521), comprenant les deux identifiants de balise (241, 242) dans l'ordre standard, en particulier l'identifiant de balise (242) du dispositif portable supplémentaire, en premier, ce qui a pour conséquence que l'entité de recherche de contact (500) est incapable de savoir que le dispositif portable (201) et l'autre dispositif portable (202) se sont rencontrés, au moyen de la valeur hachage (531) et de l'autre valeur hachage (532) étant différentes l'une de l'autre.

3. Procédé selon l'une des revendications précédentes, dans lequel les données de téléchargement (551) et les autres données de téléchargement (552) comprennent en outre également au moins une information d'horodatage, respectivement, liée à la rencontre pertinente (510) du dispositif portable (201) et du dispositif portable supplémentaire (202), dans lequel en particulier l'information d'horodatage des données de téléchargement (551)
-- dans le cas où le dispositif informatique (151) est informé d'un diagnostic, correspond à la fois au début et à la fin de la rencontre pertinente (510),
-- dans le cas où aucune information concernant un diagnostic n'est reçue par le dispositif informatique (151), correspond à un moment situé entre le début et la fin de la rencontre pertinente (510), telle que détectée par le dispositif portable (201), en particulier correspondant à la moyenne arithmétique du début et de la fin de la rencontre pertinente (510), et dans lequel en particulier l'information d'horodatage des données de téléchargement supplémentaires (552) dans le cas où le dispositif informatique (151) n'est pas informé d'un diagnostic, correspond à un moment situé entre le début et (201), correspondant en particulier à la moyenne arithmétique du début et de la fin de la rencontre pertinente (510), et dans lequel en particulier la partie d'information d'horodatage des autres données de téléchargement (552) en l'absence d'une information sur un diagnostic reçue par l'autre dispositif informatique (152), correspond à un moment situé entre le début et la fin de la rencontre pertinente (510), tel que détecté par l'autre dispositif portable (202), correspondant en particulier à la moyenne arithmétique du début et de la fin de la rencontre pertinente (510).

4. Procédé selon l'une des revendications précédentes, dans lequel - dans le cas où l'entité de recherche de contact (500) est en mesure de connaître, au moyen de l'identité de la valeur hachage de diagnostic (533) et de la valeur hachage supplémentaire (532), la rencontre pertinente (510) du dispositif portable (201) et du dispositif portable supplémentaire (202) - l'entité de recherche de contact (500) est également en mesure de savoir que l'au moins une information d'horodatage des données de téléchargement (551) et des données de téléchargement supplémentaires (552) correspondent les unes aux autres, en particulier au moyen de l'information d'horodatage des autres données de téléchargement (552) indiquant un point dans le temps entre les points dans le temps indiqués par l'information d'horodatage des données de téléchargement (551).

5. Procédé selon l'une des revendications précédentes, dans lequel à la fois les informations hachage d'entrée de diagnostic (523) et les informations hachage d'entrée standard (521, 522) comprennent, outre les deux identifiants de balise (241, 242), une information supplémentaire, l'information supplémentaire étant commune au dispositif portable (201) et au dispositif portable supplémentaire (202) mais changeant continuellement au cours du temps, l'information supplémentaire correspondant en particulier à la date actuelle ou à la semaine actuelle de la rencontre pertinente (510).

6. Procédé selon l'une des revendications précédentes, dans lequel l'identifiant personnel est ou correspond à une information relative à l'identité de la personne respective, en particulier le nom ou une autre information d'identité, ou à un pseudonyme et/ou dans lequel au moins une partie de la pluralité de personnes (100) accepte de révéler l'identifiant personnel respectif.

7. Procédé selon l'une des revendications précédentes, dans lequel, dans une situation de contact du dispositif portable (201) avec l'autre dispositif portable (202), le dispositif portable (201) est capable de déterminer de manière répétée, à différents moments, la distance par rapport à l'autre dispositif portable (202), et inversement, et de recevoir l'identifiant de balise (242) de l'autre dispositif portable par le dispositif portable (201) et l'identifiant de balise (241) du dispositif portable par l'autre dispositif portable (202), générant et stockant ainsi en particulier des données de journal.

8. Système pour révéler de manière sécurisée un identifiant personnel (541, 542) de ou lié à deux personnes, respectivement, à une entité de recherche de contact (500) d'un système ou procédé de recherche de contact basé sur la détection de rencontres pertinentes entre deux personnes, le système comprenant, outre une pluralité de dispositifs portables (200), l'entité de recherche de contact (500), et une pluralité de dispositifs informatiques (151, 152), dans lequel chacune de la pluralité de personnes (100) porte ou transporte un dispositif portable (201, 202), les dispositifs portables (201, 202) ayant ou étant associés ou attribués à, respectivement, un identifiant de balise (241, 242), les identifiants de balise (241, 242) étant diffusés de manière répétée par les dispositifs portables (201, 202), respectivement, à l'aide d'une interface de communication sans fil des dispositifs portables (201, 202), générant et/ou stockant ainsi des données de journal à partir desquelles, respectivement, des données relatives à un événement de rencontre (511) et d'autres données relatives à un événement de rencontre (512) peuvent être dérivées, comprenant respectivement les deux identifiants de balise (241, 242), un dispositif informatique (151) étant associé ou lié au dispositif portable (201), ainsi qu'un autre dispositif informatique (152) associé ou lié à l'autre dispositif portable (202), dans lequel une fonction hachage (545) est utilisée pour révéler de manière sécurisée les identifiants personnels (541, 542) dans le cas où les personnes concernées optent toutes les deux pour une telle révélation, le système étant configuré de telle sorte que :
-- une rencontre pertinente (510) du dispositif portable (201) et du dispositif portable supplémentaire (202) est détectée, indépendamment l'une de l'autre, par le dispositif portable (201) et le dispositif portable supplémentaire (202), générant et transmettant ainsi
-- les données relatives à l'événement de rencontre correspondantes (511), comprenant à la fois les identifiants de balise (241, 242) et au moins une information d'horodatage de rencontre, vers le dispositif informatique (151), et
-- les données supplémentaires relatives à l'événement de rencontre supplémentaire correspondantes (512), comprenant à la fois les identifiants de balise (241, 242) et au moins une information d'horodatage de rencontre supplémentaire, vers le dispositif informatique supplémentaire (152),
-- le dispositif informatique (151) génère des données de téléchargement (551) et l'autre dispositif informatique (152) génère d'autres données de téléchargement (552), le dispositif informatique (151) et l'autre dispositif informatique (152) transmettant les données de téléchargement respectives (551, 552) à l'entité de recherche de contact (500), dans lequel
-- le dispositif informatique (151), lorsqu'il est informé d'un diagnostic, en particulier d'un résultat de test positif, génère une valeur hachage de diagnostic (533) en calculant la fonction hachage (545) à partir d'une information hachage d'entrée de diagnostic (523), comprenant les deux identifiants de balise (241, 242) dans un ordre de diagnostic, en particulier l'identifiant de balise du dispositif portable (241) en premier, la valeur hachage de diagnostic (533) ainsi que l'identifiant personnel (541) faisant partie des données de téléchargement (551), et
-- le dispositif informatique supplémentaire (152) génère une valeur hachage supplémentaire (532) en calculant la fonction hachage (545) à partir d'une information hachage d'entrée (522) comprenant les deux identifiants de balise (241, 242) dans un ordre standard, en particulier l'identifiant de balise (241) du dispositif portable en premier, la valeur hachage supplémentaire (532) ainsi que l'identifiant personnel supplémentaire (542) faisant partie des données de téléchargement supplémentaires (552), ce qui permet à l'entité de recherche de contact (500) de savoir, au moyen de la valeur hachage de diagnostic (533) et de la valeur hachage supplémentaire (532) étant identiques, à propos de la rencontre pertinente (510) du dispositif portable (201) et de l'autre dispositif portable (202) ainsi que des identifiants personnels (541, 542).

9. Dispositifs informatiques (151, 152) et dispositifs portables (200) pour révéler de manière sécurisée un identifiant personnel (541, 542) de deux personnes, ou lié à celles-ci, respectivement, à une entité de recherche de contact (500) d'un système ou procédé de recherche de contact basé sur la détection de rencontres pertinentes entre deux personnes, dans lequel chacune de la pluralité de personnes (100) porte ou transporte un dispositif portable (201, 202), les dispositifs portables (201, 202) ayant ou étant associés ou attribués à, respectivement, un identifiant de balise (241, 242), les identifiants de balise (241, 242) étant diffusés de manière répétée par les dispositifs portables (201, 202), respectivement, à l'aide d'une interface de communication sans fil des dispositifs portables (201, 202), générant et/ou stockant ainsi des données de journal à partir desquelles, respectivement, des données relatives à un événement de rencontre (511) et d'autres données relatives à un événement de rencontre (512) peuvent être dérivées, comprenant, respectivement, les deux identifiants de balise (241, 242), un dispositif informatique (151) étant associé ou lié au dispositif portable (201), ainsi qu'un autre dispositif informatique (152) associé ou lié à l'autre dispositif portable (202), dans lequel une fonction hachage (545) est utilisée pour révéler de manière sécurisée les identifiants personnels (541, 542) dans le cas où les personnes concernées optent toutes les deux pour une telle révélation, le dispositif informatique et les dispositifs portables étant configurés de telle sorte que :
-- une rencontre pertinente (510) du dispositif portable (201) et du dispositif portable supplémentaire (202) est détectée, indépendamment l'une de l'autre, par le dispositif portable (201) et le dispositif portable supplémentaire (202), générant et transmettant ainsi
-- les données relatives à l'événement de rencontre correspondantes (511), comprenant à la fois les identifiants de balise (241, 242) et au moins une information d'horodatage de rencontre, vers le dispositif informatique (151), et
-- les données supplémentaires relatives à l'événement de rencontre correspondantes (512), comprenant à la fois les identifiants de balise (241, 242) et au moins une information d'horodatage de rencontre supplémentaire, vers le dispositif informatique supplémentaire (152),
-- le dispositif informatique (151) génère des données de téléchargement (551) et l'autre dispositif informatique (152) génère d'autres données de téléchargement (552), le dispositif informatique (151) et l'autre dispositif informatique (152) transmettant les données de téléchargement respectives (551, 552) à l'entité de recherche de contact (500), dans lequel
-- le dispositif informatique (151), lorsqu'il est informé d'un diagnostic, en particulier d'un résultat de test positif, génère une valeur hachage de diagnostic (533) en calculant la fonction hachage (545) à partir d'une information hachage d'entrée de diagnostic (523), comprenant les deux identifiants de balise (241, 242) dans un ordre de diagnostic, en particulier l'identifiant de balise (241) du dispositif portable en premier, la valeur hachage de diagnostic (533) ainsi que l'identifiant personnel (541) faisant partie des données de téléchargement (551), et
-- le dispositif informatique supplémentaire (152) génère une valeur hachage supplémentaire (532) en calculant la fonction hachage (545) à partir d'une information hachage d'entrée (522) comprenant les deux identifiants de balise (241, 242) dans un ordre standard, en particulier l'identifiant de balise (241) du dispositif portable en premier, la valeur hachage supplémentaire (532) ainsi que l'identifiant personnel supplémentaire (542) faisant partie des données de téléchargement supplémentaires (552), ce qui permet à l'entité de recherche de contact (500) de connaître, grâce à l'identité de la valeur hachage de diagnostic (533) et de la valeur hachage supplémentaire (532), la rencontre pertinente (510) du dispositif portable (201) et de l'autre dispositif portable (202) ainsi que des identifiants personnels (541, 542).

10. Programme comprenant un code de programme lisible par ordinateur qui, lorsqu'il est exécuté sur un ordinateur et/ou sur un dispositif informatique (151) et/ou sur une entité de recherche de contact (500) et/ou sur un dispositif portable (201), ou en partie sur un dispositif informatique (151) et/ou en partie sur une entité de recherche de contact (500) et/ou sur un dispositif portable (201), amène l'ordinateur et/ou le dispositif informatique (151) et/ou l'entité de recherche de contact (500) et/ou le dispositif portable (201) à exécuter un procédé selon l'une des revendications 1 à 7.

11. Support lisible par ordinateur comprenant des instructions qui, lorsqu'elles sont exécutées sur un ordinateur et/ou sur un dispositif informatique (151) et/ou sur une entité de recherche de contact (500) et/ou sur un dispositif portable (201), ou en partie sur un dispositif informatique (151) et/ou en partie sur une entité de recherche de contact (500) et/ou sur un dispositif portable (201), font que l'ordinateur et/ou le dispositif informatique (151) et/ou l'entité de recherche de contact (500) et/ou le dispositif portable (201) exécute un procédé selon l'une des revendications 1 à 7.
